# EUROPEAN PATENT APPLICATION

(11) **EP 4 575 464 A1**
(43) Date of publication of application: **25.06.2025**
(21) Application number: 24171422.9
(22) Date of filing: 19.04.2024
(51) Int. Cl.: G01N 21/51, G01N 21/64, G01N 21/77, G01N 33/543

(54) **BIOSENSING DEVICE, SAMPLE HOLDER, BIOSENSING SYSTEM AND METHOD FOR COLLECTING AND DETECTING SUPERCRITICAL ANGLE LIGHT FROM A SAMPLE**

(30) Priority: 20.12.2023 EP 23218931
(71) Applicant: Imec VZW, 3001 Leuven (BE)
(72) Inventor: JAMES SHIRLEY, Finub, 3000 Leuven (BE); STAKENBORG, Tim, 3001 Heverlee (BE); VAN ROY, Willem, 3360 Bierbeek (BE); HENRY, Olivier, 1950 Kraainem (BE); VERBRUGGEN, Bert, 3050 Oud-Heverlee (BE); VAN DORPE, Pol, 3510 Spalbeek (BE)
(74) Representative: AWA Sweden AB

(57) **Abstract**

According to an aspect there is provided a biosensing device for collecting light from a sample.

The device comprises a sensing site configured to hold a bioreceptor at a sensing surface, and to receive the sample on the sensing surface. The bioreceptor is configured to bind to an analyte in the sample. The sample light is emitted by or formed by scattering by the analyte bound to the sensing surface.

The device comprises a light guiding layer with first surface facing the sensing surface or forming the sensing surface, and having a second surface, opposite to the first surface. The layer is configured to collect supercritical angle light from the sample light of the analyte at the sensing surface, and to guide the light along an extension of the layer by total internal reflections.

The layer comprises an outcoupling region for coupling the supercritical angle light through the second surface, out of the layer.

## Description

### Technical field

The present disclosure relates to a biosensing device for collecting light from a sample, and more specifically to a biosensing device, a sample holder, a biosensing system and a method for collecting and detecting supercritical angle light from a sample.

### Background

Optical measurement methods are commonly used for studying samples in cell and molecular biology. For example, samples may be studied by optical techniques based on photoluminescence, such as fluorescence. The sample may be prepared by providing a luminescent marker, that may be selectively bound to a part of the sample that is to be analyzed, i.e. an analyte. Alternatively, an intrinsic luminescent property of the analyte in the sample may be used in exciting the analyte to emit luminescence light from the sample. Other techniques may use scattering properties of the analyte to scatter light illuminating the sample, or even chemical reactions putting the analyte in an excited state emitting chemiluminescence.

In diagnostic assays, the analyte of interest is immobilized on a bottom surface of a disposable sample holder. However, the bulk solution of the sample in the sample holder may comprise a large amount of luminescent or scattering analytes of various types. It may therefore be impossible to distinguish light from the surface bound analyte from that of the bulk solution. Consequently, if such a distinction cannot be made, the amount of the analyte of interest in the sample may not be determined. Thus, it is of importance to be able to measure specifically at the bottom surface of the sample holder.

Another challenge of using optical techniques is that the disposable sample holder needs to be properly aligned with a light source to illuminate the sample, and to a detector for detecting the light, e.g. fluorescence, from the sample. Alignment may be difficult and time consuming, which in turn may result in a low throughput of disposables and therefor also of measurements.

Yet another challenge is related to multiplexing. Multiplex immunoassays may be possible using multiple types of luminescent markers. However, this approach poses a limitation because of the number of luminescent markers that can be employed in a single assay.

Hence, there is a need in the art for further improvements related to optical measurements used in diagnostic assays.

### Summary

An objective of the present disclosure is to mitigate, alleviate or eliminate one or more of the above-identified deficiencies in the art and disadvantages singly or in any combination. These and other objectives are at least partly met by the invention as defined in the independent claims. Preferred embodiments are set out in the dependent claims.

According to a first aspect there is provided a biosensing device for collecting sample light from a sample, said device comprising:
a sensing site configured to hold a bioreceptor at a sensing surface of the sensing site, and configured to receive the sample on the sensing surface, wherein the bioreceptor is configured to bind to an analyte in the sample, wherein the sample light is emitted by or formed by scattering by the analyte bound to the bioreceptor at the sensing surface, and
a light guiding layer having a first surface facing the sensing surface of the sensing site or forming the sensing surface of the sensing site, and having a second surface, opposite to the first surface, wherein the light guiding layer is configured to collect supercritical angle light from the sample light emitted by or formed by scattering by the analyte bound to the bioreceptor at the sensing surface, and configured to guide the supercritical angle light along an extension of the light guiding layer by total internal reflections in the first and second surfaces;
wherein the light guiding layer further comprises an outcoupling region configured to couple the supercritical angle light through the second surface, out of the light guiding layer, at the outcoupling region.

By the term "sample" is here meant any physical material or matter with a particular physical and/or chemical constitution with particular characteristics. The sample may be in the form of a solid, a liquid, or a gas, or a combination thereof. However, in the present context, the sample typically has some fluid properties, such as a liquid or a gas, or a liquid comprising portions of solid material. The sample may be provided in a solution based on a liquid, such as water. By way of example, the sample may be a solution comprising a biological material, such as a bodily fluid of a human or an animal, with a solid substance such as cells.

By the term "analyte" is here meant the part of the sample that is to be analyzed, i.e. the substance of interest or the target substance. By way of example, the target substance may be a molecular species or a certain type of cell. In case a luminescent marker is added to the sample, that may selectively bind to the target substance, the term "analyte" encompasses also the combination of the luminescent marker bound to the target substance.

The biosensing device is configured to be used in analysis of the sample, with respect to the analyte. By way of example, the biosensing device may be configured to be used for measuring a concentration of the analyte in the sample.

The biosensing device may be extending in two longitudinal directions creating a substantially planar biosensing device. By way of example, the biosensing device may comprise a flat surface onto which the sensing surface of the sensing site is arranged. Alternatively, the sensing site is provided as a well on a surface of the biosensing device such that the sensing surface of the sensing site is arranged in a recess in the surface of the biosensing device.

The biosensing device may be made of either a rigid material or a flexible material, By way of example, the material may be, but is not limited to, glass or plastic. The biosensing device may be a disposable or form part of a disposable. The material may be transparent or partly transparent. In the present disclosure, the term "transparent" refers to the physical material property of allowing light to pass through the material without being substantially scattered, either at one or both surfaces of the material or when passing through the material, such that it is possible to see clearly through the material. Given as a non-limiting example, the transparency may be substantially uniform over the spectrum of visible light, such that colors viewed through the transparent material are essentially unaltered.

The biosensing device may be provided as a standalone unit. Given only as a non-limiting example, the biosensing device may be similar in size and shape to a glass slide for microscopy. The biosensing device may have a rectangular shape, but may alternatively have a square shape, a circular shape, an elliptical shape or any other shape suitable for collection of a sample.

Alternatively, the biosensing device may form part of a sample holder further comprising side walls, such that the biosensing device forms a bottom part of the sample holder. The side walls may be extending from a surface of the biosensing device, and along a perimeter of the biosensing device, such that the side walls surround the sensing site. By the present arrangement, the biosensing device and the side walls may together define a volume for receiving and holding the sample. The sensing site is arranged on the surface of the biosensing device facing the volume, such that the sensing surface of the sensing site can receive the sample in the volume.

By way of further example, the biosensing device may be configured as a component to be assembled with components such as side walls to form a sample holder.

The biosensing device further comprises a light guiding layer. The light guiding layer may be extending in a plane in two longitudinal directions creating a substantially planar light guiding layer. Given as a non-limiting example, the light guiding layer may be provided in the form of a slab waveguide. The light guiding layer comprises a first surface and a second surface, opposite to the first surface. The first surface and the second surface may be parallel to the plane of extension of the light guiding layer. Typically the first surface forms the sensing surface of the sensing site, such that the first surface receives, and may thus be in direct physical contact with, the sample. However, it is conceivable that first surface may be provided with an additional layer of another material, for example for protecting the light guiding layer and/or for providing good attachment properties for the bioreceptor to the sensing surface. In such a case, the additional layer may form the sensing surface, and the first surface of the light guiding layer may be facing the sensing surface of the sensing site. In such a case, the first surface may be parallel to the sensing surface. Typically, the light guiding layer may extend parallel to the extension of the biosensing device.

The second surface is facing away from the sensing surface. The second surface may be configured to face a detector configured to detect light.

By way of example, the light guiding layer may be made of silicon nitride.

Light from the sample, sample light, may be emitted by or formed by scattering by the analyte in the sample. Typically the refractive index of the sample solution is lower than the refractive index of a materials of which the light guiding layer of the biosensing device is made. Thus, sample light from the analyte in the sample bulk, passing across the interface between the sample solution and the light guiding layer of the biosensing device will be subject to an increase in refractive index, and consequently be refracted to a refraction angle being smaller than the angle of incidence. In other words, the light having propagated across the interface will have a refraction angle at or below the critical angle of refraction of the interface. This is referred to as undercritical angle light. Since the undercritical angle light has an angle at or below the critical angle, the light may not be guided by the light guiding layer by total internal reflections and thus the light may pass through the light guiding layer out of the second surface.

However, the analyte bound to the bioreceptors at the surface may be at or below a distance of one wavelength from the first surface of the interface. Light of the analyte at such short distance from the first surface of the interface may be passed across the interface at angles larger than the critical angle. This is referred to as supercritical angle light. Supercritical angle light has an angle above the critical angle and may thus be subject to total internal reflection at the first and second surfaces of the light guiding layer.

Thus by the present arrangement, the light guiding layer is configured to collect the supercritical angle light from the analyte at the sensing surface, and to guide the supercritical angle light along the extension of the light guiding layer by total internal reflections in the first and second surfaces.

It serves to mention that, in this context the term "light" should be allowed a broad interpretation, not limited to visible electromagnetic radiation. Rather, the term "light" may also include for example ultra-violet light and infra-red light.

The light guiding layer further comprises an outcoupling region. The outcoupling region is configured to couple the supercritical angle light through the second surface, out of the light guiding layer, at the outcoupling region. The outcoupling region is laterally displaced from a region of the light guiding layer beneath the sensing site in which supercritical angle light is collected by the light guiding layer. By the present arrangement, the supercritical angle light from the analyte at the sensing surface will be collected and guided along the extension of the light guiding layer, whereas undercritical angle light may propagate through the light guiding layer. In this manner, the supercritical angle light may be spatially separated from the undercritical angle light, by guiding the supercritical angle light away from the center of the sensing site. Upon reaching the outcoupling region, the supercritical light is redirected towards the second surface and coupled through the second surface, out of the light guiding layer. This spatial separation allows for detection of the supercritical angle light to be made, without interference from undercritical angle light.

By way of example, the outcoupling region may be arranged circularly symmetric with respect to the sensing site. By such an arrangement, the supercritical angle light may be outcoupled through the second surface in the shape of a ring at the second surface, whereas the undercritical angle light may exit the second surface in the shape of a circle surrounded by the ring of supercritical angle light.

An advantage is that a biosensing device configured for a simplified light collection scheme may be provided. When using the biosensing device in a system with a detector, the simplified light collection scheme allows the biosensing device to be aligned with respect to the detector, and to an illuminating light source if applicable, in a short time. Thus, switching between different biosensing devices may be done in a short time, allowing for a high throughput.

Another advantage is that by coupling the supercritical angle light through the second surface, out of the light guiding layer, at the outcoupling region, the use of a plurality of sensing sites on the biosensing device is enabled in a simple manner. Supercritical angle light from the plurality of sensing sites may be outcoupled from the light guiding layer through spatially separated outcoupling regions, thereby allowing separate detection of a plurality of supercritical angle light from the plurality of sensing sites on the same biosensing device.

Yet another advantage is that a biosensing device with low light losses may be provided. The arrangement may provide efficient light collection by the use of the light guiding layer collecting and guiding the light by total internal reflections. Further, the biosensing device allows for improved signal-to-noise ratio.

According to an embodiment, the sensing site is further configured to receive illumination light through the sample on the sensing surface for illuminating the analyte thereby exciting the analyte, causing the analyte to form the sample light at the sensing surface by emitting photoluminescence.

Thus, the sample light may be photoluminescence, such as for example fluorescence or phosphorescence. The analyte may be excited by the illumination light by absorption of light leaving the analyte in higher energy state. After a short time, the analyte may release the excess energy by spontaneous emission of light, i.e. fluorescence or phosphorescence. Typically the emitted light is wavelength shifted with respect to the illumination light. Typically at least some of the emitted light is shifted towards longer wavelengths, with respect to the illumination light.

Note that the analyte originally present in the sample, may be photoluminescent by itself when subjected to illumination light. Alternatively, a luminescent marker may be added to the sample, that may selectively bind to the target substance, the combination of which forms a photoluminescent analyte. By way of example, luminescent markers used for fluorescence measurements may be referred to a fluorophores.

An advantage with this embodiment is that a versatile biosensing device may be provided. Photoluminescence may provide selective measurements of a large number of analytes to be made. This is due to the excitation relying on the illumination light having a wavelength matching an energy transmission in the analyte in order for the light absorption to take place. By the present arrangement, by selecting the wavelength of illumination light to match the analyte or marker, sample light from the analyte may be generated, and at the same time light from other substances in the sample may be avoided.

According to an embodiment, the sensing site is further configured to receive illumination light through the sample on the sensing surface for illuminating the analyte, causing the analyte to form the sample light at the sensing surface by scattering of the illumination light.

Put differently, the illumination light may be scattered by the analyte in the sample, thereby forming the sample light. By the term "scattered light" is here meant elastically scattered light, thus light scattered such that the direction and/or phase may change but the energy of the photons, and thus the wavelength of the light is substantially unchanged, apart from a slight Doppler shift that may result from the movement of the sample. In other words, the scattering process does not involve any net energy transfer between the light and the scattering sample, in terms of e.g. change in electronic energy states of the atoms or molecules in the sample. Given as non-limiting examples, the elastic scattering may be, but is not limited to, Rayleigh or Mie scattering.

According to an embodiment, the sample undergoes a chemical reaction to excite the analyte causing the analyte to form the sample light at the sensing surface by emitting chemiluminescence.

Thus, the chemical reaction may form the analyte in an excited state, resulting in emission of the sample light when excess energy of the analyte is released.

According to an embodiment, the outcoupling region comprises a grating coupler configured to redirect the supercritical angle light in the light guiding layer towards the second surface of the light guiding layer, so as to couple the supercritical angle light through the second surface, out of the light guiding layer, at the outcoupling region.

An advantage with this embodiment is that a compact and efficient manner of redirecting the supercritical angle light in the light guiding layer towards the second surface of the light guiding layer may be provided.

According to an embodiment, the grating coupler is configured to redirect the supercritical angle light in the light guiding layer in dependence of a wavelength of the supercritical angle light, such that an outcoupling angle at which the supercritical angle light is coupled through the second surface out of the light guiding layer is dependent on the wavelength.

Put differently, supercritical angle light of different wavelengths may be coupled through the second surface out of the light guiding layer in different outcoupling angles, and thus in different directions. When using the biosensing device in a system with a detector comprising a plurality of light sensitive elements, the biosensing device may be arranged with respect to the detector so that different angular components of the supercritical angle light, and thus different wavelengths, or wavelength bands, of the supercritical angle light, may be detected by different light sensitive elements. By the present arrangement, multi-spectral measurements at the same sensing site may be enabled.

Multi-spectral measurements may be used in a number of different applications. By way of example, fluorescence measurements may involve multiple wavelengths. For example, if more than one molecular species is excited, their respective fluorescence may be emitted at different wavelengths. By detecting the supercritical angle light at different outcoupling angles by different light sensitive elements on the detector, multi-fluorescence measurements may be enabled on the same sensing site.

Given as a non-limiting example, for measuring the concentration of an analyte in the sample, a known concentration of a control substance may be mixed into the sample. The sensing site of the biosensing device may further hold a control bioreceptor configured to bind to the control substance, in addition to holding the bioreceptor configured to bind to the analyte. Fluorescence from the control substance may be emitted at a different wavelength, or wavelength range, as compared to the fluorescence emitted by the analyte. The supercritical angle fluorescence from the control substance at the sensing surface may thus be collected by the light guiding layer and guided along the light guiding layer by total internal reflection. The supercritical angle fluorescence from the control substance may further be coupled through the second surface, out of the light guiding layer, at the outcoupling region, at an outcoupling angle different from the outcoupling angle of the supercritical angle fluorescence from the analyte. This enables detection of the supercritical angle fluorescence from the control substance by different light sensitive elements of the detector, than the supercritical angle fluorescence of the analyte. By the present arrangement, multi-fluorescence detection at the same sensing site is enabled. A response curve of the control substance may be acquired which may be used for calibration of the measurement of the analyte. In this manner, a more accurate quantification of the analyte may be provided. Further, this approach may also correct for degradation of the bioreceptors that may occur during long term storage.

According to an embodiment, the outcoupling region comprises a reflector arranged at a surface configured to face the sample.

According to an embodiment, the reflector extends in a plane parallel to the extension of the light guiding layer.

According to an embodiment, the reflector is configured to reflect supercritical angle light towards the second surface.

By way of example, in case supercritical angle light being guided in the light guiding layer may exit the light guiding layer in a direction towards the sample volume, the reflector may reflect the supercritical angle light back towards the second surface such that the supercritical angle light may be coupled out through the second surface. Thus, when the biosensing unit is arranged onto a detector in a biosensing system, the supercritical angle light may be coupled out through the second surface to reach the detector.

An advantage with this embodiment is that improved outcoupling of the supercritical angle light from the light guiding layer may be provided, which in turn may improve the efficiency of light detection.

According to an embodiment, the reflector is configured to prevent light, emitted by or formed by scattering by the analyte not bound to the bioreceptor at the sensing surface, from reaching the outcoupling region.

By way of example, light emitted by or formed by scattering by the analyte in the bulk solution of the sample, may be directed towards the outcoupling region of the biosensing device. The reflector is arranged at the surface configured to face the sample such that the reflector may reflect such light back into the bulk solution of the sample. By the present arrangement, undercritical angle light from the bulk solution may be prevented from reaching the outcoupling region.

An advantage with this embodiment is that the separation of supercritical angle light and undercritical angle light may be further enhanced.

According to an embodiment, the reflector comprises at least one metal layer.

By way of example, the reflector may be provided by a surface coating.

According to an embodiment, the light guiding layer has a refractive index in the range of 1.4 to 4, and preferably 1.7 to 2.1.

Typically the refractive index of the materials of which the light guiding layer is made is higher than the refractive index of the sample solution. Thus, sample light from the analyte in the sample bulk, passing across the interface between the sample solution and the light guiding layer will be subject to an increase in refractive index, and consequently be refracted to a refraction angle being smaller than the angle of incidence. By way of example, the light guiding layer may be made of silicon nitride. Silicon nitride has a refractive index of about 1.9.

An advantage with this embodiment is that a light guiding layer having a refractive index within said range may be especially suitable for use together with a water based sample.

According to an embodiment, the sensing site forms part of a plurality of sensing sites of the biosensing device,
wherein each sensing site of the plurality of sensing sites is configured to hold a bioreceptor at a sensing surface of the sensing site, and configured to receive the sample on the sensing surface, wherein the bioreceptor is configured to bind to an analyte in the sample, wherein the sample light is emitted by or formed by scattering by the analyte bound to the bioreceptor at the sensing surface of each respective sensing site.

An advantage with this embodiment is that it allows spatially separated detection of supercritical angle light from the plurality of sensing sites on the same biosensing device. This, in turn, provides a biosensing device allowing for high throughput supercritical angle light measurements to be made.

Another advantage is that the biosensing device with a plurality of sensing sites may be provided with sufficient simplicity allowing for alignment to an illumination and detection system to be made in a short time. The present advantage provides further increase of throughput.

According to an embodiment, the light guiding layer is a common light guiding layer for the plurality of sensing sites, the light guiding layer being configured to collect supercritical angle light from the sample light emitted by or formed by scattering by the analyte bound to the bioreceptor at the sensing surface of each respective sensing site, and configured to guide the supercritical angle light along an extension of the light guiding layer by total internal reflections in the first and second surfaces.

It should be understood that the supercritical angle light from the respective sensing sites may be collected and guided by different portions of the light guiding layer, such that spatial separation between the supercritical angle light from the respective sensing sites is maintained.

An advantage with the present embodiment is that a biosensing device with a plurality of sensing sites may be easily manufactured. During the manufacturing process, manufacturing one common light guiding layer for all the sensing sites of the biosensing device, is easier and less time consuming than manufacturing a separate light guiding layer for each of the plurality of sensing sites.

According to an embodiment, the light guiding layer further comprises a plurality of outcoupling regions, wherein each outcoupling region of the plurality of outcoupling regions is associated with a mutually unique sensing site of the plurality of sensing sites, and wherein each outcoupling region is configured to couple the supercritical angle light through the second surface of the respective sensing site, out of the light guiding layer, at the outcoupling region.

According to an embodiment, different sensing sites of the plurality of sensing sites are configured to hold different types of bioreceptors configured to bind to different analytes in the sample.

The present embodiment enables measurements of several different analytes of the sample to be made, by the same biosensing device. Since the sensing sites are spatially separated, the embodiment enables measurement of several different analytes to be made by spatial separating. Thus supercritical angle light from the respective analytes may be individually measured without interference from light from the other analytes.

It is conceivable that different sensing sites of the plurality of sensing sites receive different samples. Hence, different sensing sites of the plurality of sensing sites may be configured to hold different types of bioreceptors configured to bind to different analytes in the different samples.

An advantage with this embodiment is that a biosensing device for a high degree of multiplexing may be provided. Thus, measurement of several different analytes on the same biosensing device may be enabled.

Another advantage is that, if a luminescent marker is used to bind to one or more substances to form luminescence analytes, the same luminescent marker may be used for several different analytes. The different analytes are bound to the respective bioreceptors in different sensing sites, and the signals of supercritical light from the respective analytes are thus spatially separated. Therefore there is no need to separate the different analytes spectrally, i.e. by providing luminescence at different wavelengths. Moreover, with the same luminescent marker used for all of the plurality of analytes, the same illumination light may be used for all of the plurality of analytes, and more precisely the illumination light for all of the plurality of sensing sites and analytes may have the same wavelength.

According to an embodiment, each sensing site of the plurality of sensing sites comprises a shield configured to prevent sample light from the sensing site to reach adjacent sensing sites.

An advantage with this embodiment is that improved spatial separation between the light at the plurality of sensing sites may be provided. This is turn may improve the signal to noise ratio of the respective measurements.

According to a second aspect there is provided a sample holder for collecting sample light from a sample, the sample holder comprising:
the biosensing device according to the first aspect, and
side walls extending from a surface of the biosensing device, and along a perimeter of the biosensing device, such that the side walls surround the sensing site;
wherein the biosensing device and the side walls together define a volume for receiving the sample, and wherein the sensing site is arranged on the surface of the biosensing device facing the volume, such that the sensing surface of the sensing site can receive the sample in the volume.

In case the biosensing device comprises a single sensing site, the side walls of the sample holder surround the single sensing site. However, it should be understood that in case the biosensing device comprises a plurality of sensing sites, the side walls may surround not only the sensing site but rather the plurality of sensing sites.

In this manner, a sample holder may be provided configured to receive and hold a sample in the volume defined by the biosensing device and the side walls, such that the plurality of sensing surfaces of the plurality of sensing sites may receive the sample thereon.

It is conceivable that, as an alternative, a microfluidic channel may lead the sample to the sensing site or the plurality of sensing sites. By way of example, the biosensing device may form part of a microfluidic channel such that the sample may be made to flow through the biosensing device. In such an arrangement, the volume receiving the sample is defined by an integral part of the biosensing device and thus of the microfluidic channel.

According to a third aspect there is provided a biosensing system comprising:
a sample holder according to the second aspect; and
a detector comprising a plurality of light sensitive elements, wherein each light sensitive element of the plurality of light sensitive elements is configured to generate an electric signal dependent on an intensity of light incident onto the respective light sensitive element;
wherein the detector is arranged to receive the supercritical angle light being coupled out of the light guiding layer.

By the term "detector" is here meant any unit or device onto which a plurality of separate light sensitive elements are arranged such that they may individually detect the light intensity impinging onto the respective light sensitive element, and in response thereof produce an electrical signal. The light sensitive elements on the detector may be arranged along one or more rectilinear lines. By way of example, a detector may be, but is not limited to, a charge-coupled device (CCD) and complementary metal oxide semiconductor (CMOS).

By way of example, the plurality of light sensitive elements may be arranged in a plane of the detector. By way of further example, the plane may be a planar surface of the detector.

As previously described, the biosensing device of the sample holder may separate the supercritical angle light from the undercritical angle light. Thus by arranging the biosensing device of the sample holder onto or in parallel with the planar surface of the detector, the supercritical angle light may be detected by separate light sensitive elements, from the undercritical angle light. It should be realized that the biosensing device of the sample holder may be arranged above the detector, either onto and thus in physical contact with the detector, or above but not in physical contact with the detector.

An advantage with this embodiment is that the supercritical angle light, from the analyte at the sensing surface, may be detected without interference from the undercritical angle light, from the bulk solution of the sample. Thus, surface bound analyte may be monitored separately from the rest of the bulk solution.

Another advantage is that the present arrangement enables a compact and simple optical set-up for collection of light towards the detector. In particular, no bulky optical components, such as lenses or mirrors for collecting the supercritical angle light are necessary.

By way of example, traditional optics may be omitted such that the biosensing system may form a lens-free imaging biosensing system. An advantage is that cost and size of the image acquisition of the biosensing system may be significantly reduced, while still maintaining high sensitivity.

Another advantage is that the lens-free imaging biosensing system may provide a large field of view compared to systems using traditional optics. The present arrangement allows for the use of a large biosensing device comprising a large number of sensing sites. In the manner described above, measurements may be performed on the several hundreds to thousands of proteins in the sample.

It serves to mention that the biosensing system may be configured such that the supercritical angle light and the undercritical angle light are separated and received by the detector onto separate light sensitive elements, thereby allowing the surface to bulk analyte ratio to be determined.

According to an embodiment, the biosensing system further comprises:
a light source configured to generate illumination light; and
wherein the sample holder is arranged such that the sensing site receives the illumination light through the sample on the sensing surface.

By the term "light source" is here meant any unit, device and/or element at which light is generated. By way of example, the light source may be, but is not limited to a laser, a laser diode, a light emitting diode, an incandescent light source, a fluorescent light source, or a combination thereof.

The light source may be configured to generate illumination light to excite the analyte to emit the sample light. Alternatively, the light source may be configured to generate illumination light to be scattered by the analyte, thereby forming the sample light.

By the present arrangement, it is possible to provide illumination light through the bulk solution of the sample. As sample light from the bulk solution is not collected and guided by the light guiding layer, the supercritical angle light at the sensing surface may be separated from undercritical angle light from the bulk, and may thus still be detected. The light source and the detector may be arranged on separate sides of sample holder, which implies that a simple set-up may be provided, e.g. no waveguides may be required.

An advantage is that the present arrangement may provide a biosensing system allowing the sample holder to be arranged and aligned with respect to the light source and the detector in a short time. Thus, switching between different sample holders may be done in a short time, allowing for a high throughput.

According to an embodiment, the light source is configured to provide polarized illumination light.

By polarized illumination light is here typically meant linearly polarized illumination light. However, it is conceivable that the light source may be configured to provide also other types of polarized illumination light. By way of example, the illumination light from the light source may be polarized by an illumination polarization filter arranged in the light path of the illumination light from the light source. Alternatively, the light source may generate light of a certain polarization, such as a laser generating linearly polarized light.

According to an embodiment, the biosensing system further comprises a detection polarization filter arranged between the biosensing device of the sample holder and the detector.

The detection polarization filter may be arranged such that the polarization direction allowed to pass through the detection polarization filter is aligned with the polarization direction of the polarized illumination light. Thus, light with the polarization direction of the polarized illumination light may pass through the detection polarization filter to reach the detector, whereas light of other polarization directions may be blocked by the filter, thereby being prevented from reaching the detector.

An advantage with this embodiment is that the separation of supercritical angle light and undercritical angle light may be further enhanced. This in turn may allow the system to further differentiate between surface bound analytes and analytes of the bulk solution.

By way of example, a detection polarization filter in combination with polarized illumination light may be used in a fluorescence collection system. Immobilized surface analytes may preserve their polarization in relation to the excitation polarization to a higher degree than free-floating analytes of the bulk solution. This may allow for selectively measure the fluorescence contribution of surface bound analytes even in the region below the center of the sensing site, where undercritical angle light is otherwise expected to be the major part of the light exiting through the second surface. By the present arrangement, further exclusion of fluorescence from the bulk solution may be provided, which may result in better sensitivity.

According to an embodiment, the biosensing device comprises a plurality of sensing sites, and
wherein the detector is a common detector for the plurality of sensing sites, the detector being arranged such that supercritical angle light from different sensing sites of the plurality of sensing sites is incident onto different light sensitive elements of the plurality of light sensitive elements, thereby allowing separate detection of the supercritical angle light from the plurality of sensing sites, respectively.

The plurality of sensing sites may be configured to hold the same type of bioreceptor configured to bind to the same type of analyte in the sample. Alternatively, different sensing sites of the plurality of sensing sites may be configured to hold different types of bioreceptors configured to bind to different analytes in the sample.

An advantage is that a sample holder with a plurality of sensing sites may enable multiple detections to be made simultaneously, thereby providing a high throughput.

Another advantage is that a biosensing system for a high degree of multiplexing may be provided. Thus, measurement of several different analytes on the same biosensing device may be enabled.

Yet another advantage is that, if a luminescent marker is used to bind to substances in the sample to form luminescence analytes, the same luminescent marker may be used for several different analytes. The different analytes may be bound to the respective bioreceptors in different sensing sites, and the signals of supercritical light from the respective analytes are thus spatially separated. Therefore there is no need to separate the different analytes spectrally, i.e. by providing luminescence at different wavelengths.

Yet another advantage is that, with the same luminescent marker used for all of the plurality of analytes, the same illumination light may be used for all of the plurality of analytes, and more precisely the illumination light for all of the plurality of sensing sites and analytes may have the same wavelength. Thus, the same illumination light wavelength may be used to excite the luminescent markers bound to the plurality of sensing sites. This provides a simplified illumination set-up.

According to an embodiment, the biosensing system may be configured to be loaded with a plurality of sample holders, and to advance the sample holder from the plurality of sample holders to a sample position.

The sample holders of the plurality of sample holders may be advanced, one by one, to the sample position. At the sample position the sample holder may be aligned with the detector, and the light source if applicable, such that the detector may detect supercritical angle light from the sensing site or the plurality of sensing sites of the sample holder. Prior to advancing a consecutive sample holder to the sample position, the sample holder located at the sample position may be advanced away from the sample position, making space for the consecutive sample holder.

By way of example, the advancement of the sample holder may be provided by one or more conveyer belts, rotating plates, rotating wheels or a combination thereof.

It is conceivable that the biosensing system may comprise a plurality of sample positions, such that the sample holders of the plurality of sample holders may be advanced to different sample positions of the plurality of sample positions.

According to a fourth aspect there is provided a method for collecting sample light from a sample, by a biosensing device, the method comprising:
receiving, on a sensing surface of a sensing site of the biosensing device, the sample, wherein the sensing site is configured to hold a bioreceptor at the sensing surface of the sensing site;
binding, to the bioreceptor, an analyte in the sample, wherein the sample light is emitted by or formed by scattering by the analyte bound to the bioreceptor at the sensing surface;
collecting, by a light guiding layer, supercritical angle light from the sample light emitted by or formed by scattering by the analyte bound to the bioreceptor at the sensing surface, wherein the light guiding layer has a first surface facing the sensing surface of the sensing site or forming the sensing surface of the sensing site, and has a second surface, opposite to the first surface;
guiding, in the light guiding layer, the supercritical angle light along an extension of the light guiding layer by total internal reflections in the first and second surfaces; and
coupling the supercritical angle light through the second surface, out of the light guiding layer, at an outcoupling region of the light guiding layer.

Effects and features of the second, third and fourth aspects are largely analogous to those described above in connection with the first aspect. Embodiments mentioned in relation to the first aspect are largely compatible with the second, third and fourth aspects. It is further noted that the disclosure relates to all possible combinations of features unless explicitly stated otherwise.

Other objectives, features and advantages of the present disclosure will appear from the following detailed description, from the attached claims as well as from the drawings.

### Brief descriptions of the drawings

The above, as well as additional objects, features and advantages of the present disclosure, will be better understood through the following illustrative and non-limiting detailed description, with reference to the appended drawings. In the drawings like reference numerals will be used for like elements unless stated otherwise.
Fig. 1 schematically illustrates a cross-sectional side-view of a biosensing device for collecting supercritical angle light from a sample.
Fig. 2A schematically illustrates a top-view of a biosensing device comprising a plurality of sensing sites, for collecting supercritical angle light from a sample.
Fig. 2B schematically illustrates a sample holder for collecting supercritical angle light from a sample.
Fig. 3A schematically illustrates a biosensing system for detection of supercritical light from a sample.
Fig. 3B illustrates a diagram of a detected signal of supercritical angle light as detected by a detector.
Fig. 3C schematically illustrates a cross-sectional side-view of a biosensing system using polarized illumination light and a detection polarization filter.
Fig. 3D schematically illustrates a cross-sectional side-view of a biosensing system comprising a grating coupler configured to redirect light in in dependence of the wavelength of the light.
Fig. 4 schematically illustrates a biosensing system that may be loaded with a plurality of sample holders and configured to advance sample holders.
Fig. 5 illustrates a schematic block diagram shortly summarizing the method for collecting sample light from a sample, by a biosensing device.

### Detailed description

In cooperation with attached drawings, the technical contents and detailed description of the present inventive concept are described thereinafter according to a preferable embodiment, being not used to limit the claimed scope. This inventive concept may be embodied in many different forms and should not be construed as limited to the embodiments set forth herein; rather, these embodiments are provided for thoroughness and completeness, and fully convey the scope of the inventive concept to the skilled person.

Hereinafter, a biosensing device will be described in the context of collection of supercritical angle fluorescence light. However, it should be realized that fluorescence is merely an example of supercritical angle light, and that the description is relevant also for other types of supercritical angle light, such as other supercritical angle photoluminescence light, supercritical angle scattered light and supercritical angle chemiluminescence light.

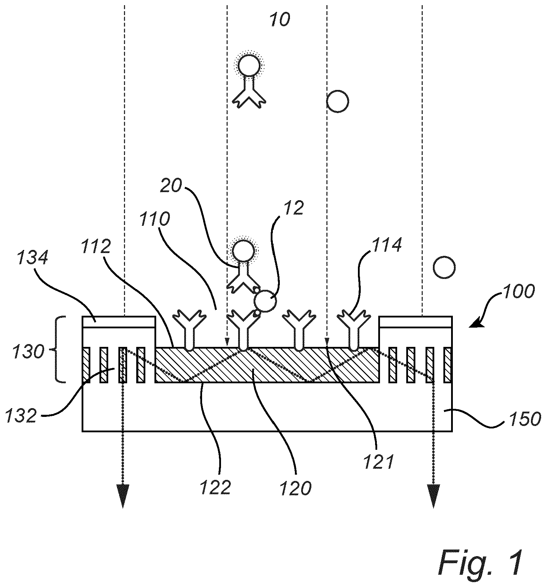

Fig. 1 schematically illustrates a cross-sectional side-view of a biosensing device 100 for collecting sample light from a sample 10.

The sample 10 may be a water based solution comprising a biological material, such as a bodily fluid of a human or an animal. The sample 10 comprises an analyte 12, i.e. a substance of interest or the target substance to be measured. By way of example, the target substance may be a molecular species or a certain type of cell. In the present example of photoluminescence, the analyte 12 does not comprise any intrinsic fluorescent properties. Therefore, a fluorescent marker 20 may be added to the sample 10, wherein the fluorescent marker 20 may selectively bind to the analyte 12. By binding the marker 20 to the analyte 12, excitation of the analyte 12, or more specifically the marker 20, may be enabled, thereby allowing induced fluorescence measurements to be made of the analyte 12.

The biosensing device 100 comprises a substrate 150. The substrate is typically made of a transparent material such as glass or plastic. By way of example, the substrate may be made of Silicon Oxide.

The biosensing device 100 comprises a sensing site 110 with a sensing surface 112. When in use, the sensing surface 112 typically faces upwards. Thus, the sample 10 may be held on top of the biosensing device 100, such that the sensing site 110 may receive the sample 10 on the sensing surface 112. The sensing site 110 may hold a bioreceptor 114 at a sensing surface 112. The bioreceptor 114 is configured to bind to the analyte 12 in the sample 10, such that the analyte 12 is bound to the sensing surface 112 by the bioreceptor 114.

In the present example, the sensing site 110 is configured to receive illumination light through the sample 10 on the sensing surface 112 for illuminating the analyte 12. By the present arrangement with the fluorescent marker 20 bound to the analyte 12, the analyte 12 may be excited, causing the analyte 12 to form sample light by emitting photoluminescence, and in the present example more specifically fluorescence. It is conceivable that sample light in the form of fluorescence may be emitted by the analyte 12 in the entire bulk solution of the sample 10. More specifically, sample light in the form of fluorescence may be emitted by the analyte 12 bound to the bioreceptor 114 at the sensing surface 112.

The biosensing device 100 further comprises a light guiding layer 120. The light guiding layer 120 is arranged in a plane of the biosensing device 100. The light guiding layer 120 has a first surface 121 forming the sensing surface 112 of the sensing site 110, and thus faces the sample 10. Further, the light guiding layer 120 has a second surface 122, opposite to the first surface 121.

The refractive index of a material of which the light guiding layer 120 is made may be selected to be higher than the refractive index of the solution of the sample 10. Thus, sample light from the analyte 12 in the sample 10 bulk, passing across the interface between the sample 10 solution and the light guiding layer 120, i.e. the first surface 121, will be subject to an increase in refractive index. Consequently, the refraction angle is smaller than the angle of incidence, and will be at or below the critical angle of refraction of the interface. This undercritical angle light, or undercritical angle fluorescence, may pass through the light guiding layer out of the second surface below the sensing site 110. By way of example, the light guiding layer may be made of silicon nitride, having a refractive index of about 1.9.

However, the analyte 12 bound to the bioreceptors 114 at the sensing surface 112 may be at a short enough distance from the first surface 121 to be passed across the interface at angles larger than the critical angle as supercritical angle light, or supercritical angle fluorescence. The light guiding layer 120 is configured to collect supercritical angle light from the sample light emitted by the analyte 12 at the sensing surface 112. The supercritical angle light has an angle above the critical angle and may thus be subject to total internal reflections at the first surface 121 and the second surface 122 of the light guiding layer. By the present arrangement, the light guiding layer 120 may guide the supercritical angle light along the extension of the light guiding layer 120 by total internal reflections in the first 121 and second surfaces 122. In this manner, the supercritical angle light may be spatially separated from the undercritical angle light, by guiding the supercritical angle light away from the center of the sensing site 110.

The supercritical angle light collected by the light guiding layer 120 may be guided to an outcoupling region 130. The outcoupling region 130 is configured to couple the supercritical angle light through the second surface 122, out of the light guiding layer 120, at the outcoupling region 130. By way of example, the outcoupling region 130 may comprise a grating coupler 132 configured to redirect the supercritical angle light in the light guiding layer 120 towards the second surface 122 of the light guiding layer 120. By the present arrangement, the supercritical angle light may be coupled out of the light guiding layer 120. Since the supercritical angle light and the undercritical angle light are spatially separated, the supercritical angle light and thus light from the analyte 12 bound to the bioreceptor 114 at the sensing surface 112 may be detected, without interference from undercritical angle light from the bulk.

Optionally, the outcoupling region 130 may be provided with a reflector 134 arranged at a surface configured to face the sample 10. The reflector 134 may improve the outcoupling of light through the second surface 122 by reflecting supercritical angle light that might otherwise propagate towards the sample volume, back towards the second surface such that the supercritical angle light may be coupled out through the second surface. Alternatively or additionally, the reflector 134 may prevent light, emitted by the analyte 12 not bound to the bioreceptor 114 at the sensing surface 112, from reaching the outcoupling region 130. The present arrangement may thus enhance the separation of supercritical angle light and undercritical angle light.

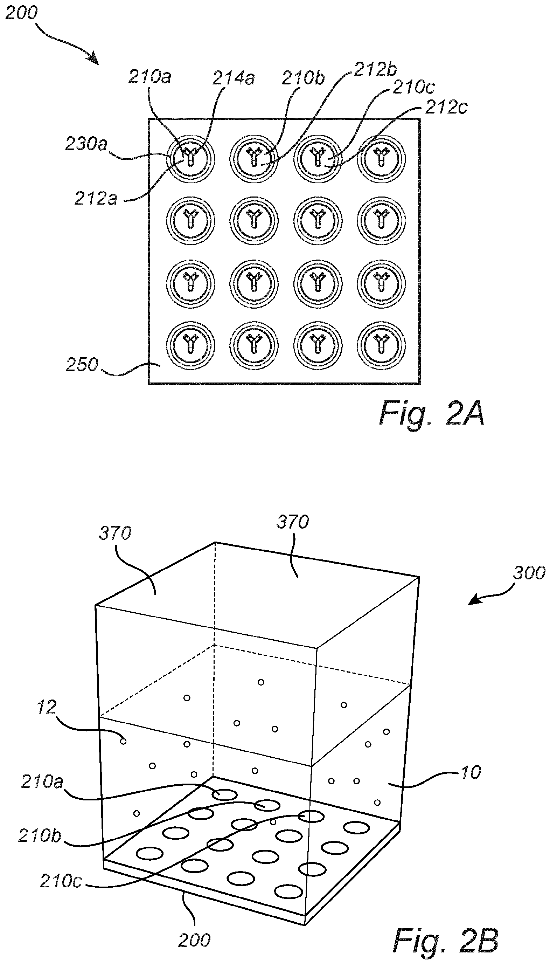

Fig. 2A schematically illustrates a top-view of a biosensing device 200 for collecting sample light from a sample 10. The biosensing device 200 comprises a plurality of sensing sites 210a, 210b, 21 0c. The sensing sites 210a, 210b, 210c of biosensing device 200 share some of the features with the sensing site 110 of biosensing device 100 described in relation to Fig. 1, the details of which are not repeated here.

The biosensing device 200 comprises a common substrate 250 for all of the plurality of sensing sites 21 0a, 21 0b, 210c. The sample 10 may be held on top of the substrate 250 of the biosensing device 100, such that the plurality of sensing sites 21 0a, 21 0b, 210c may receive the sample 10 on the respective sensing surfaces 212a, 212b, 212c. For simplicity the present description will be made in relation to sensing site 210a only, however it should be realized that similar reasonings are relevant for all of the plurality of sensing sites 210a, 210b, 210c.

Each sensing site 210a, 210b, 210c of the plurality of sensing sites may hold a bioreceptor 214a at a sensing surface 212a of the sensing site 210a, 210b, 210c. In the present example, different sensing sites 210a, 210b, 210c of the plurality of sensing sites are configured to hold different types of bioreceptors 214a. The different types of bioreceptors 214a are configured to bind to different types of analytes (not illustrated here) in the sample 10. The present arrangement enables a plurality of different types of analytes in the sample 10 to be detected by the same biosensing device 200. Since the different types of analytes are bound to different locations on the biosensing device 200, their respective sample light are spatially separated, allowing for simultaneous detection to be made. It serves to mention that, as an alternative each sensing site 210a, 210b, 210c of the plurality of sensing sites may hold the same type of bioreceptor 214a, 210b, 210c. This may allow multiple detections of the same type of analyte to be made simultaneously using the same biosensing device 200.

The biosensing device 200 comprises a common light guiding layer (not illustrated here) for the plurality of sensing sites 210a, 210b, 210c. The light guiding layer may extend along the plane of the biosensing device 200 so as to provide light guiding layer below each of the plurality of sensing sites 210a, 210b, 210c. The light guiding layer may be configured to collect supercritical angle light, i.e. supercritical angle fluorescence, from the sample light emitted by the respective analytes at each respective sensing site 210a, 210b, 210c. Further, the light guiding layer may be configured to guide the supercritical angle light collected at the respective sensing sites 210a, 210b, 210c along an extension of the light guiding layer by total internal reflections.

The light guiding layer of the biosensing device 200 comprises a plurality of outcoupling regions 230a. Thus, for each respective sensing site 210a, 210b, 210c there is an associated outcoupling region 230a. In the present example, each outcoupling region 230a is arranged circularly symmetric in the light guiding layer, around the center of the associated sensing site 210a, 210b, 210c. The respective outcoupling regions 230a are configured to couple the supercritical angle light of the respective sensing site 210a, 210b, 210c, through the second surface, out of the light guiding layer, at the respective outcoupling regions 230a. By the present arrangement, supercritical angle light from the respective sensing sites 210a, 210b, 210c are outcoupled at different locations of the biosensing device 200, and thus spatially separated. This enables detection of sample light from the different sensing sites 210a, 210b, 210c and thus from different types of analytes in the sample 10 to be made separately and simultaneously, by using the same biosensing device 200.

Although not illustrated here, the outcoupling region 230a may optionally be provided with a reflector arranged at a surface facing the sample 10. The reflector may improve the outcoupling of light and/or may prevent unwanted light from the bulk solution of the sample 10, from reaching the outcoupling region 230a.

It serves to mention that, although not illustrated here, each of the sensing sites may further comprise a shield configured to prevent sample light from the sensing site to reach adjacent sensing sites. By way of example, said shields may be arranged in the light guiding layer or in the substrate. By this arrangement, cross-talk between the different sensing sites 210a, 210b, 210c may be reduced or eliminated.

Fig. 2B schematically illustrates a sample holder 300 for collecting sample light from a sample 10.

The sample holder 300 comprises the biosensing device 200 as described in relation to Fig. 2A. However, it should be understood that the sample holder 300 may alternatively comprise any biosensing device, such as the biosensing device 100 as described in relation to Fig. 1.

The biosensing device 200 may function as a bottom plate of the sample holder 300.

The sample holder 300 further comprises side walls 370 extending from a surface of the biosensing device 200, and along a perimeter of the biosensing device 200. In this manner, the side walls 370 surround the sensing sites 210a, 210b, 210c of the biosensing device. By the present arrangement, the biosensing device 200 and the side walls 370 together define a volume for receiving the sample 10. Further, the biosensing device 200 is arranged such that the plurality of sensing sites 210a, 210b, 210c faces the volume holding the sample 10. Thus, the sensing surfaces of the respective sensing sites 210a, 210b, 210c can receive the sample 10 in the volume.

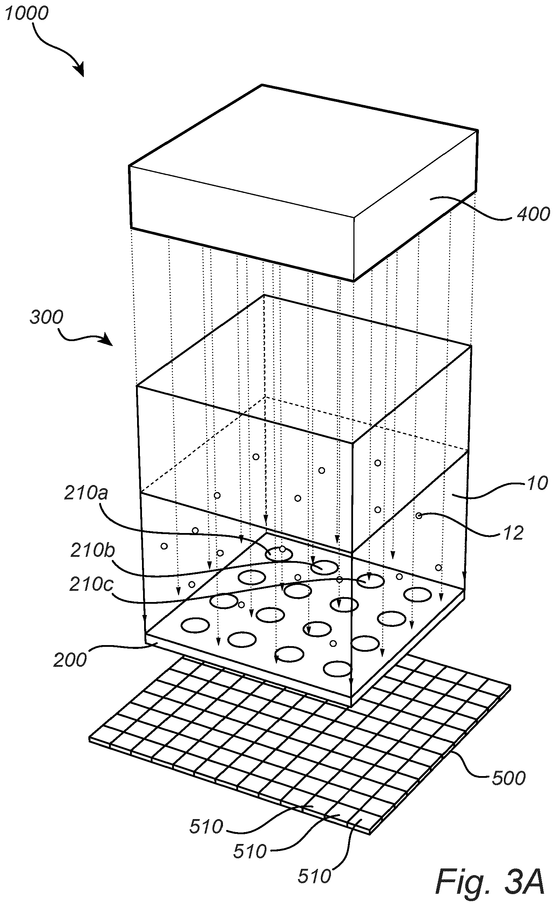

Fig. 3A schematically illustrates a biosensing system 1000. In the present example, the biosensing system 1000 is a system for detection of supercritical fluorescence light. However, it should be realized that a biosensing system may optionally be configured for detection of other types of sample light, such as light formed by scattering or chemiluminescence.

The biosensing system 1000 comprises a sample holder 300, as described in relation to Fig. 2B having a biosensing device 200 as described in relation to Fig. 2A. However, it should be understood that the sample holder 300 may alternatively comprise any biosensing device, such as the biosensing device 100 as described in relation to Fig. 1. The sample holder 300 can hold a sample 10 in the volume of the sample holder 300.

The biosensing system 1000 further comprises a light source 400. The light source 400 is configured to generate illumination light. The light source 400 is arranged such that when the sample holder 300 is arranged in a sample position, the sample 10 in the sample holder may be illuminated by the illumination light from the light source 400. Put differently, the sample holder 300 may be arranged in the biosensing system 1000 such that the plurality of sensing sites 210a, 210b, 210c receive the illumination light through the sample 10 on the respective sensing surfaces. The analyte 12 may be excited by illumination of the sample 10, causing the analyte 12 to form sample light by emitting fluorescence. At each respective sensing site 210a, 210b, 210c, supercritical angle fluorescence light, from the respective analytes at the respective sensing surface, may be collected by the light guiding layer (not illustrated here). The supercritical angle fluorescence light, may be guided in the light guiding layer and outcoupled from the light guiding layer at the respective outcoupling regions, thereby being spatially separated from the undercritical angle fluorescence light.

The biosensing system 1000 further comprises a detector 500 comprising a plurality of light sensitive elements 510. The detector 500 is arranged in the biosensing system 1000 to receive the supercritical angle light being coupled out of the light guiding layer at the bottom of the sample holder 300. Since the biosensing device 200 of the sample holder 300 comprises a plurality of sensing sites 210a, 210b, 210c, the detector 500 may be a common detector 500 for the plurality of sensing sites 210a, 210b, 210c.

The detector 500 may therefore be arranged such that supercritical angle light from different sensing sites 210a, 210b, 210c of the plurality of sensing sites is incident onto different light sensitive elements 510 of the plurality of light sensitive elements 510. By the present arrangement, separate detection of the supercritical angle light from the plurality of sensing sites 210a, 210b, 210c, respectively, may be provided.

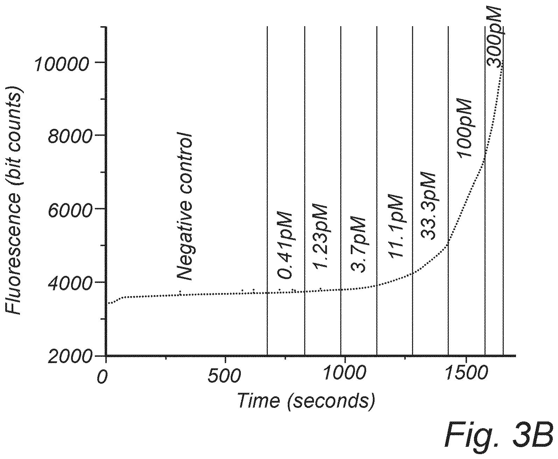

Fig. 3B illustrates a diagram of a detected signal of supercritical angle light as detected by the detector. As mentioned, the supercritical angle light is a result of the analyte bound to the bioreceptors at the sensing surface. As more and more analyte is bound to the bioreceptors at the surface over time, the detected signal gradually increases over time. Since the signal from the detector is in relation to the light incident onto the respective light sensitive elements, the signal from the detector may be used to quantitatively determine the amount of analyte at the sensing surface of the sample holder. Further, from the detected signal the concentration of the analyte in the sample may be quantitatively determined.

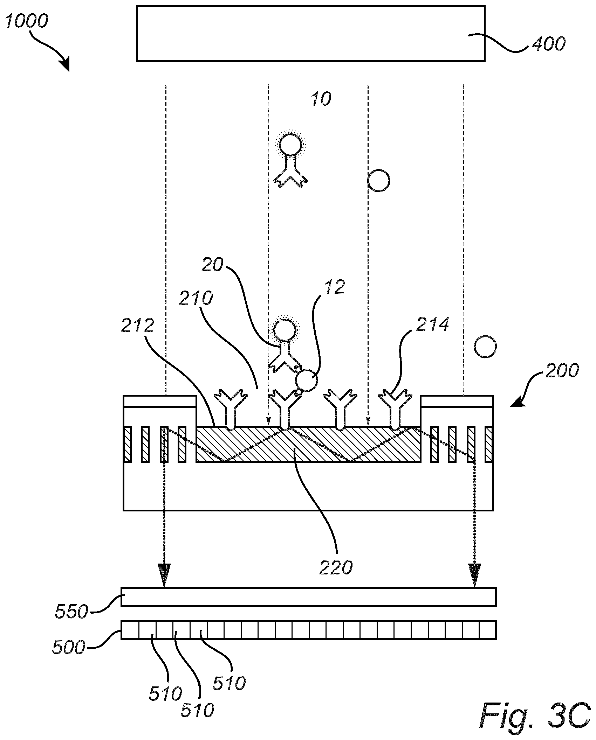

Fig. 3C schematically illustrates a cross-sectional side-view of the arrangement of the light source 400, a biosensing device 200, and the detector 500 of the biosensing system 1000.

Optionally, the light source 400 may be configured to provide polarized illumination light. Polarized illumination light may be provided by using a laser as the light source 400, or by arranging an illumination polarization filter in the light path of the illumination light from the light source 400.

Optionally, the biosensing system 1000 may further comprise a detection polarization filter 550. The detection polarization filter 550 may be arranged between the sensing site 210 of the biosensing device 200 and the detector 500.

The detection polarization filter 550 may be configured to allow light of a specific polarization direction to pass the detection polarization filter 550, and to block light of other polarization directions, thereby preventing the other polarizations from reaching the detector 500. The detection polarization filter 550 may be arranged to be aligned with the polarization direction of the polarized illumination light. Thus, light with the polarization direction of the polarized illumination light may pass through the detection polarization filter 550 to reach the detector 500, whereas other light may be blocked.

On the sensing surface 212 of the sensing site 210, analytes 12 may be bound to the bioreceptors 114. These immobilized surface analytes 12 may preserve their polarization in relation to the excitation polarization to a higher degree than free-floating analytes of the bulk solution. Thus, the fluorescence emitted by the surface bound analytes 12 may have the same polarization direction as the polarized illumination light from the light source 400. On the other hand, fluorescence from analytes in the bulk solution may have any polarization direction. In this manner, selective measurements of the fluorescence of surface bound analytes 12 may be enabled, even in the region below the center of the sensing site 210, by further exclusion of fluorescence from the bulk solution.

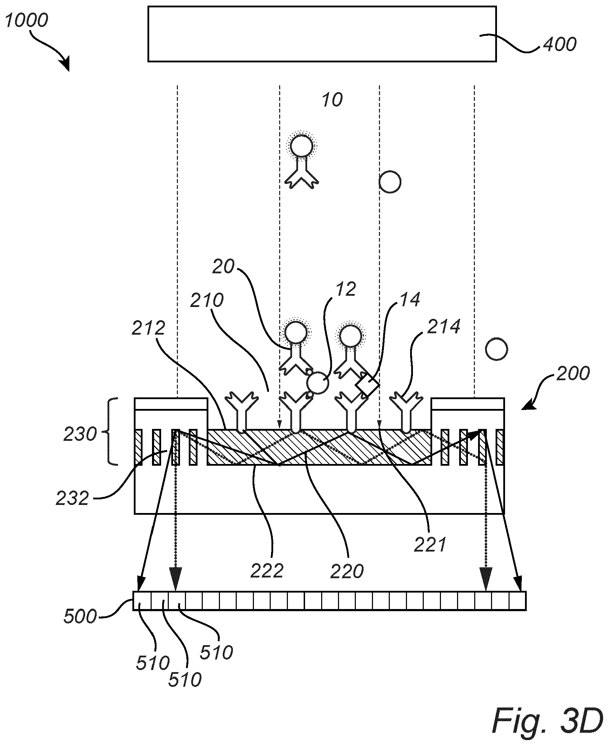

Fig. 3D schematically illustrates a further cross-sectional side-view of the arrangement of the light source 400, a biosensing device 200, and the detector 500 of the biosensing system 1000.

Optionally, a known concentration of a control substance 14 may be mixed info the sample 10, in order to improve the accuracy of the measurements of the analyte 12. The control substance 14 may be bound to a control bioreceptor 214 at the sensing surface 212 of the sensing site 210, which is the present example is the same bioreceptor 214 that also binds the analyte 12. However, it is conceivable that the control bioreceptor may alternatively be a bioreceptor of a different type.

The control substance 14, in the present example in combination with a fluorescent marker 20, may be excited by the illumination light from the light source 400. Fluorescence from the control substance 14 may be emitted at a different wavelength, as compared to the fluorescence emitted by the analyte 12. Supercritical angle fluorescence from the surface bound control substance 14 may be collected by the light guiding layer 220 and guided along the light guiding layer 220 to the outcoupling region 230.

Optionally, the grating coupler 232 may be configured to redirect the supercritical angle fluorescence in the light guiding layer in dependence of the wavelength of the supercritical angle fluorescence. In this manner, the outcoupling angle at which the supercritical angle fluorescence is coupled through the second surface 222 out of the light guiding layer 220 may be dependent on the wavelength. The supercritical angle fluorescence from the control substance 14 may thus be coupled out of the light guiding layer 220 at a different outcoupling angle than the supercritical angle fluorescence from the analyte 12.

The biosensing device 200 may be arranged with respect to the detector 500 so that different angular components of the supercritical angle light, and thus different wavelengths of the supercritical angle light may be detected by different light sensitive elements 510. By the present arrangement, detection of the supercritical angle fluorescence from the control substance 14 may be detected by different light sensitive elements 510 of the detector 500, than the supercritical angle fluorescence of the analyte 12, enabling simultaneous and independent detection of supercritical angle fluorescence from the analyte 12 and the control substance 14. Since the concentration of the control substance 14 is known, the acquired response curve of the control substance 14 may optionally be used for calibration of the measurement of the analyte 12. By the present arrangement a more accurate quantification of the analyte 12 may be provided.

Although the multi-spectral approach described in relation to Fig. 3D and the approach using polarized illumination light and a detection polarization filter described in relation to Fig. 3C are here illustrated as two separate options, it should be understood that the two options may alternatively also be combined.

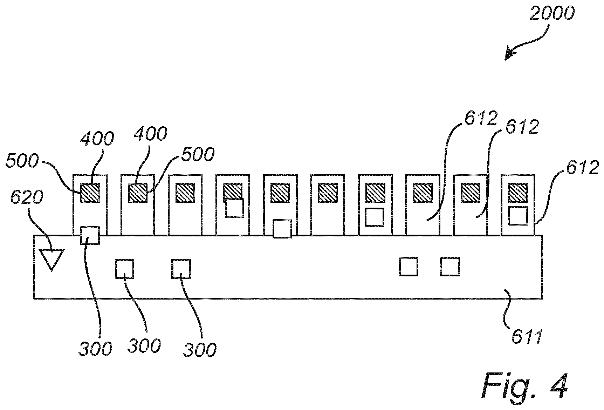

Fig. 4 schematically illustrates a biosensing system 2000 that may be loaded with a plurality of sample holders 300. The biosensing system 2000 may be configured to advance a sample holder 300 from the plurality of sample holders 300 to a sample position. In the present example, the biosensing system comprises a plurality of sample positions. Further, the biosensing system 1000 in the present example is a system for detection of supercritical fluorescence light. However, it should be realized that a biosensing system may optionally be configured for detection of other types of sample light, such as light formed by scattering or chemiluminescence.

The sample holders 300 may be of the type as described in relation to Fig. 2B having a biosensing device 200 as described in relation to Fig. 2A, the details of which are not repeated here.

The sample holders 300 may be advanced, one by one, by a sample loading conveyor belt 611. In the present example, the sample holders 300 are advanced to sample injection position where a sample injector 620 may inject the sample into the respective sample holders 300. Given as a non-limiting example, the sample may in the form of a liquid solution. A liquid solution may be easily injected into the sample holders 300.

Once the sample has been injected into the sample holder 300, the sample holder is advanced by the sample loading conveyor belt 611 towards an available sample position. A measurement conveyor belt 612 may pick up the sample holder 300 from the sample loading conveyor belt 611 and advance the sample holder 300 to the available sample position.

At each sample position of the plurality of sample positions, the biosensing device 2000 comprises a light source 400 arranged above the sample position to illuminate the sample once the sample holder 300 is in the sample position. The analyte may be excited by illumination of the sample, causing the analyte to form sample light by emitting fluorescence. At each respective sensing site, supercritical angle fluorescence light, from the respective analytes at the respective sensing surface, may be collected by the respective light guiding layer, and guided to the respective outcoupling region where the supercritical angle fluorescence light is outcoupled.

At each sample position of the plurality of sample positions, the supercritical angle fluorescence light biosensing device 2000 further comprises a detector 500 with a plurality of light sensitive elements. The detector 500 is arranged below the sample position to receive the supercritical angle light being coupled out at the bottom of the sample holder 300.

The detector 500 may be arranged such that supercritical angle light from different sensing sites is incident onto different light sensitive elements of the detector 500, thereby providing separate detection of the supercritical angle fluorescence light from the plurality of sensing sites, respectively.

Once the detection of supercritical angle fluorescence light is completed, the measurement conveyor belt may transport the sample holder 300 away from the sample position, making the sample position available again for a new sample holder 300.

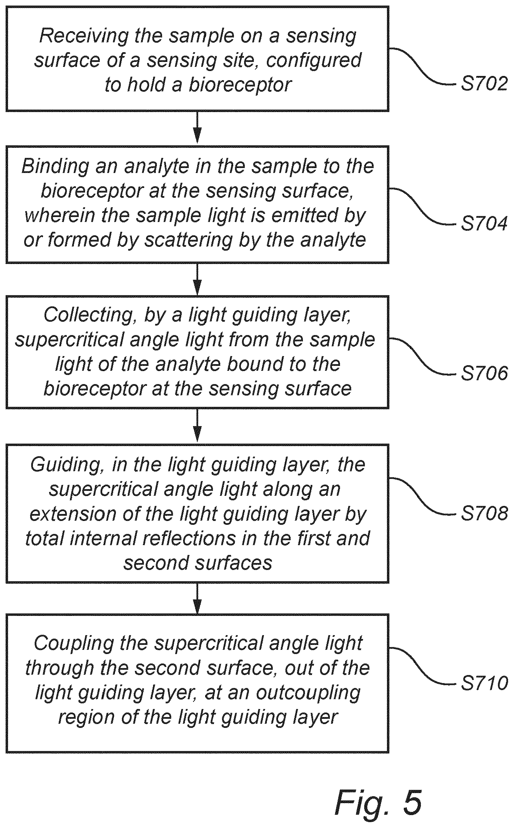

Fig. 5 illustrates a schematic block diagram shortly summarizing the method for collecting sample light from a sample, by a biosensing device. It should be understood that the steps of the method, although listed in a specific order herein, may be performed in any order suitable.

The method may comprise receiving S702, on a sensing surface of a sensing site of the biosensing device, the sample. The sensing site may be configured to hold a bioreceptor at the sensing surface of the sensing site.

The method may comprise binding S704, to the bioreceptor, an analyte in the sample. The sample light may be emitted by or formed by scattering by the analyte bound to the bioreceptor at the sensing surface.

The method may comprise collecting S706, by a light guiding layer, supercritical angle light from the sample light emitted by or formed by scattering by the analyte bound to the bioreceptor at the sensing surface. The light guiding layer may have a first surface facing the sensing surface of the sensing site or forming the sensing surface of the sensing site, and may have a second surface, opposite to the first surface.

The method may comprise guiding S708, in the light guiding layer, the supercritical angle light along an extension of the light guiding layer by total internal reflections in the first and second surfaces.

The method may comprise coupling S710 the supercritical angle light through the second surface, out of the light guiding layer, at an outcoupling region of the light guiding layer.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A biosensing device (100, 200) for collecting sample light from a sample (10), said device comprising:
a sensing site (110, 210) configured to hold a bioreceptor (114, 214) at a sensing surface (112, 212) of the sensing site (110, 210), and configured to receive the sample (10) on the sensing surface (112, 212), wherein the bioreceptor (114, 214) is configured to bind to an analyte (12) in the sample (10), wherein the sample light is emitted by or formed by scattering by the analyte (12) bound to the bioreceptor (114, 214) at the sensing surface (112, 212), and
a light guiding layer (120) having a first surface (121) facing the sensing surface (112) of the sensing site (110, 210) or forming the sensing surface (112, 212) of the sensing site (110, 210), and having a second surface (122), opposite to the first surface (121), wherein the light guiding layer (120) is configured to collect supercritical angle light from the sample light emitted by or formed by scattering by the analyte (12) bound to the bioreceptor (114, 214) at the sensing surface (112, 212), and configured to guide the supercritical angle light along an extension of the light guiding layer (120) by total internal reflections in the first (121) and second surfaces (122);
wherein the light guiding layer (120) further comprises an outcoupling region (130, 230) configured to couple the supercritical angle light through the second surface (122), out of the light guiding layer (120), at the outcoupling region (130, 230).

2. The biosensing device (100, 200) of claim 1, wherein the sensing site (110, 210) is further configured to receive illumination light through the sample (10) on the sensing surface (112, 212) for illuminating the analyte (12) thereby exciting the analyte (12), causing the analyte (12) to form the sample light at the sensing surface (112, 212) by emitting photoluminescence.

3. The biosensing device (100, 200) according to any one of claims 1 or 2, wherein the outcoupling region (130, 230) comprises a grating coupler (132) configured to redirect the supercritical angle light in the light guiding layer (120) towards the second surface (122) of the light guiding layer (120), so as to couple the supercritical angle light through the second surface (122), out of the light guiding layer (120), at the outcoupling region (130, 230).

4. The biosensing device (100, 200) according to any one of the preceding claims, wherein the outcoupling region (130, 230) comprises a reflector arranged at a surface configured to face the sample (10).

5. The biosensing device (100, 200) according to claim 4, wherein the reflector extends in a plane parallel to the extension of the light guiding layer (120).

6. The biosensing device (100, 200) according to any one of claims 4 or 5, wherein the reflector is configured to reflect supercritical angle light towards the second surface (122).

7. The biosensing device (100, 200) according to any one of claims 4 to 6, wherein the reflector is configured to prevent light, emitted by or formed by scattering by the analyte (12) not bound to the bioreceptor (114, 214) at the sensing surface (112, 212), from reaching the outcoupling region (130, 230).

8. The biosensing device (200) according to any one of the preceding claims, wherein the sensing site (210) forms part of a plurality of sensing sites (210) of the biosensing device (200),
wherein each sensing site (210) of the plurality of sensing sites (210) is configured to hold a bioreceptor (214) at a sensing surface (212) of the sensing site (210), and configured to receive the sample (10) on the sensing surface (212), wherein the bioreceptor (214) is configured to bind to an analyte (12) in the sample (10), wherein the sample light is emitted by or formed by scattering by the analyte (12) bound to the bioreceptor (214) at the sensing surface (212) of each respective sensing site (210).

9. The biosensing device (200) according to claim 8, wherein the light guiding layer is a common light guiding layer for the plurality of sensing sites (210), the light guiding layer being configured to collect supercritical angle light from the sample light emitted by or formed by scattering by the analyte (12) bound to the bioreceptor (214) at the sensing surface (212) of each respective sensing site (210), and configured to guide the supercritical angle light along an extension of the light guiding layer by total internal reflections in the first and second surfaces.

10. The biosensing device (200) according to any one of claims 8 or 9, wherein different sensing sites (210) of the plurality of sensing sites (210) are configured to hold different types of bioreceptors (214) configured to bind to different analytes (12) in the sample (10).

11. A sample holder (300) for collecting sample light from a sample (10), the sample holder comprising:
the biosensing device (100, 200) according to any one of the preceding claims, and
side walls (370) extending from a surface of the biosensing device (100, 200), and along a perimeter of the biosensing device (100, 200), such that the side walls (370) surround the sensing site (110, 210);
wherein the biosensing device (100, 200) and the side walls (370) together define a volume for receiving the sample (10), and wherein the sensing site (110, 210) is arranged on the surface of the biosensing device (100, 200) facing the volume, such that the sensing surface (112, 212) of the sensing site (110, 210) can receive the sample (10) in the volume.

12. A biosensing system (1000, 2000) comprising:
A sample holder (300) according to claim 11; and
a detector (500) comprising a plurality of light sensitive elements (510), wherein each light sensitive element (510) of the plurality of light sensitive elements (510) is configured to generate an electric signal dependent on an intensity of light incident onto the respective light sensitive element (510);
wherein the detector (500) is arranged to receive the supercritical angle light being coupled out of the light guiding layer (120).

13. The biosensing system (1000, 2000) according to claim 12, further comprising:
a light source (400) configured to generate illumination light; and
wherein the sample holder (300) is arranged such that the sensing site (110, 210) receives the illumination light through the sample (10) on the sensing surface (112, 212).

14. The biosensing system (1000, 2000) according to any one of claims 12 or 13, wherein the biosensing device (200) comprises a plurality of sensing sites (210), and
wherein the detector (500) is a common detector (500) for the plurality of sensing sites (210), the detector (500) being arranged such that supercritical angle light from different sensing sites (210) of the plurality of sensing sites (210) is incident onto different light sensitive elements (510) of the plurality of light sensitive elements (510), thereby allowing separate detection of the supercritical angle light from the plurality of sensing sites (210), respectively.

15. A method for collecting sample light from a sample, by a biosensing device, the method comprising:
receiving (S702), on a sensing surface of a sensing site of the biosensing device, the sample, wherein the sensing site is configured to hold a bioreceptor at the sensing surface of the sensing site;
binding (S704), to the bioreceptor, an analyte in the sample, wherein the sample light is emitted by or formed by scattering by the analyte bound to the bioreceptor at the sensing surface;
collecting (S706), by a light guiding layer, supercritical angle light from the sample light emitted by or formed by scattering by the analyte bound to the bioreceptor at the sensing surface, wherein the light guiding layer has a first surface facing the sensing surface of the sensing site or forming the sensing surface of the sensing site, and has a second surface, opposite to the first surface;
guiding (S708), in the light guiding layer, the supercritical angle light along an extension of the light guiding layer by total internal reflections in the first and second surfaces; and
coupling (S710) the supercritical angle light through the second surface, out of the light guiding layer, at an outcoupling region of the light guiding layer.
